(19) Europäisches Patentamt
European Patent Office
Office européen des brevets

(11) Veröffentlichungsnummer: **0 000 198**
**B1**

(12) **EUROPÄISCHE PATENTSCHRIFT**

(21) Anmeldenummer: **78100251.4**

(22) Anmeldetag: **28.06.78**

(51) Int. Cl.[3]: **C 07 D 239/46,**
A 01 N 47/18

# (54) N,N-Dialkyl-O-pyrimidinyl-carbaminsäureester, Verfahren zu ihrer Herstellung und ihre Verwendung als Insektizide

(30) Priorität: **05.07.77 DE 2730273**

(43) Veröffentlichungstag der Anmeldung:
**10.01.79 Patentblatt 79/01**

(45) Bekanntmachung des Hinweises auf die Patenterteilung:
**23.07.80 Patentblatt 80/15**

(84) Benannte Vertragsstaaten:
**BE CH DE FR GB NL SE**

(56) Entgegenhaltungen:
**CHEMICAL ABSTRACTS, Vol. 87 (1977) p. 526, 527, 39.530u 551 329**

(73) Patentinhaber: **Bayer AG**
**Zentralbereich Patente, Marken und Lizenzen**
**D - 5090 Leverkusen 1, Bayerwerk (DE)**

(72) Erfinder: **Maurer, Fritz, Dr.**
**Roeberstrasse 8**
**D - 5600 Wuppertal 1 (DE)**
**Hammann, Ingeborg, Dr.**
**Belfortstrasse 9**
**D - 5000 Köln 1 (DE)**
**Homeyer, Bernhard, Dr.**
**Obere Strasse 28**
**D - 5090 Leverkusen 3 (DE)**

**EP 0 000 198 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

## N,N-Dialkyl-O-pyrimidinyl-carbaminsäureester, Verfahren zu ihrer Herstellung und ihre Verwendung als Insektizide

Die vorliegende Erfindung betrifft neue N,N-Dialkyl-O-pyrimidinyl-carbaminsäureester, Verfahren zu ihrer Herstellung sowie ihre Verwendung als Insektizide.

Es ist bereits bekannt, daß N,N-Dimethyl-O-pyrimidinylcarbaminsäureester, z.B. N,N-Dimethyl-O-(2-isopropyl-6-methyl-pyrimidin(4)yl)- oder -O-(2-dimethylamino-6-methylpyrimidin(4)yl)-carbaminsäureester, insektizide Eigenschaften haben (vergleiche USA-Patentschrift 2 694 712 und Britisches Patent 1 181 657).

Es wurden nun die neuen N,N-Dialkyl-O-pyrimidinyl-carbaminsäureester der Formel (I)

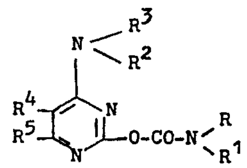

(I)

gefunden,
worin
R bis $R^3$ für gleiches oder verschiedenes Alkyl,
$R^4$ für Wasserstoff, Alkyl, Alkylthio oder Halogen und
$R^5$ für Wasserstoff oder Alkyl stehen.

Diese neuen Verbindungen zeichnen sich durch insektizide Eigenschaften aus.

Weiterhin wurde gefunden, daß die N,N-Dialkyl-O-pyrimidinyl-carbaminsäureester (I) erhalten werden, wenn man 2-Hydroxy-4-dialkylamino-pyrimidine der Formel (II)

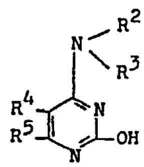

(II)

in welcher
$R^2$ bis $R^5$ die oben angegebene Bedeutung haben,

a) gegebenenfalls in Form der Alkali- oder Erdalkalisalze oder gegebenenfalls in Gegenwart eines Säureakzeptors mit N,N-Dialkyl-carbaminsäurehalogeniden der Formel (III)

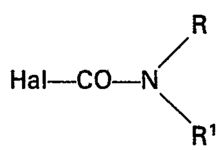

(III)

in welcher
R und $R^1$ die oben angegebene Bedeutung haben und
Hal für Halogen, vorzugsweise Chlor, steht,
gegebenfalls in Gegenwart eines Verdünnungsmittels umsetzt, oder

b) gegebenfalls in Gegenwart eines Verdünnungsmittels mit Phosgen umsetzt und anschließend mit einem Amin der allgemeinen Formel IV

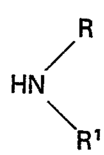

(IV)

in welcher
R und $R^1$ die oben angegebene Bedeutung haben,
gegebenfalls in Gegenwart eines Säureakzeptors umsetzt.

Überraschenderweise besitzen die erfindungsgemäßen N,N-Dialkyl-O-pyrimidinyl-carbaminsäureester eine bessere insektizide Wirkung als die aus der Literatur vorbekannten Verbindungen analoger Konstitution und gleicher Wirkungsrichtung. Die Stoffe gemäß vorliegender Erfindung stellen somit eine echte Bereicherung der Technik dar.

Verwendet man beispielsweise bei Verfahrensvariante a) 2-Hydroxy-4-dimethylamino-5-chlor-

pyrimidin und N,N-Diäthylcarbaminsäurechlorid als Ausgangsmaterialien, so kann der Reaktionsverlauf durch das folgende Formelschema wiedergegeben werden:

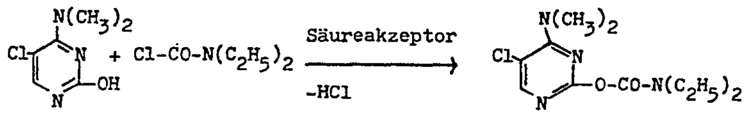


Verwendet man beispielsweise bei Verfahrensvariante b) 2-Hydroxy-4-dimethylamino-pyrimidin Phosgen und Dimethylamin als Ausgangsmaterialien, kann der Reaktionsverlauf durch das folgende Formelschema wiedergegeben werden

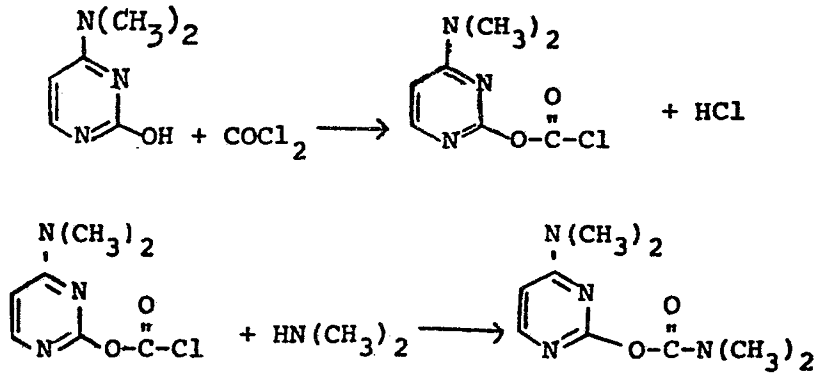

Die zu verwendenden Ausgangsstoffe sind durch die Formeln (II), (III) und (IV) allgemein definiert. Vorzugsweise stehen darin jedoch

R bis $R^3$ für gleiches, geradkettiges oder verzweigtes Alkyl mit 1 bis 6 Kohlenstoffatomen, insbesondere Methyl oder Äthyl,

$R^4$ für Wasserstoff, Chlor, geradkettiges oder verzweigtes Alkyl bzw. Alkylthio mit 1 bis 4 Kohlenstoffatomen je Alkyl- bzw. Alkylthiorest, insbesondere 1 oder 2 Kohlenstoffatome und

$R^5$ für Wasserstoff oder geradkettiges oder verzweigtes Alkyl mit 1 bis 4, insbesondere 1 oder 2, Kohlenstoffatome.

Die als Ausgangsstoffe zu verwendenden 2-Hydroxy-4-dialkylamino-pyrimidine(II) können nach literaturbekannten Verfahren hergestellt werden; für den Fall, daß, $R^4$ für Wasserstoff, Alkyl oder Halogen steht, aus den entsprechenden 2-Halogenverbindungen durch Verseifung und für den Fall, daß $R^4$ für Alkylthio steht, aus den entsprechenden in 5-Stellung unsubstituierten 2-Hydroxy-pyrimidinen, Dimethyldisulfid und Sulfurylchlorid gegebenenfalls in Gegenwart eines Lösungsmittels.

Als Beispiele für die 2-Hydroxy-4-dialkylamino-pyrimidine (II) seien im einzelnen genannt;
2-Hydroxy-4-dimethylamino-pyrimidin,
2-Hydroxy-5-diäthylamino-pyrimidin,
2-Hydroxy-4-dimethylamino-5-chlor-pyrimidin,
2-Hydroxy-4-dimethylamino-5-methyl-pyrimidin,
2-Hydroxy-4-dimethylamino-5-methylthio-pyrimidin,
2-Hydroxy-4-dimethylamino-5-äthylthio-pyrimidin,
2-Hydroxy-4-dimethylamino-5,6-dimethyl-pyrimidin,
2-Hydroxy-4-dimethylamino-5-methylthio-6-methyl-pyrimidin,
2-Hydroxy-4-dimethylamino-5-chlor-6-methyl-pyrimidin,
2-Hydroxy-4-diäthylamino-5-chlor-pyrimidin,
2-Hydroxy-4-diäthylamino-5-methyl-pyrimidin,
2-Hydroxy-4-diäthylamino-5-methylthio-pyrimidin,
2-Hydroxy-4-diäthylamino-5-äthylthio-pyrimidin,
2-Hydroxy-4-diäthylamino-5,6-dimethyl-pyrimidin,
2-Hydroxy-4-diäthylamino-5-methylthio-6-methyl-pyrimidin,
2-Hydroxy-4-diäthylamino-5-chlor-6-methyl-pyrimidin.

Die weiterhin als Ausgangsstoffe zu verwendenden N,N-Dialkylcarbaminsäurehalogenide (III) sowie die Amine (IV) sind literaturbekannt und nach üblichen Methoden gut herstellbar. Als Beispiele dafür seien im einzelnen genannt:

N,N-Dimethyl- und N,N-Diäthyl-carbaminsäurechlorid sowie Dimethylamin und Diäthylamin.

Die Verfahren zur Herstellung der erfindungsgemäßen Verbindungen werden bevorzugt unter Mitverwendung geeigneter Lösungsmittel oder Verdünnungsmittel durchgeführt. Als solche kommen praktisch alle inerten organischen Solventien in Frage. Hierzu gehören insbesondere aliphatische und aromatische, gegebenenfalls chlorierte, Kohlenwasserstoffe, wie Benzol, Toluol, Xylol, Benzin,

Methylenchlorid, Chloroform, Tetrachlorkohlenstoff, Chlorbenzol, oder Ather, z.B. Diäthyl- und Dibutyläther, Dioxan, ferner Ketone, beispielsweise Aceton, Methyl-äthyl-, Methyl-isopropyl- und Methyl-isobutylketon, außerdem Nitrile, wie Aceto- und Propionitril.

Als Säureakzeptoren können alle üblichen Säurebindemittel Verwendung finden. Besonders bewährt haben sich Alkalicarbonate und -alkoholate, wie Natrium- und Kaliumcarbonat, -methylat bzw. -äthylat, ferner aliphatische, aromatische oder heterocyclische Amine, beispielsweise Trimethylamin, Triäthylamin, Dimethylanilin, Dimethylbenzylamin und Pyridin.

Die Reaktionstemperatur kann innerhalb eines größeren Bereichs variiert werden. Im allgemeinen arbeitet man zwischen 0 und 100°C, vorzugsweise bei 30 bis 80°C.

Die Umsetzung läßt man im allgemeinen bei Normaldruck ablaufen.

Zur Durchführung der Verfahrensvariante a) setzt man die Carbaminsäurehalogenidkomponente vorzugsweise in 10 bis 30%igem Überschuß ein. Die Reaktionspartner werden meist in einem organischen Lösungsmittel in Gegenwart eines Säureakzeptors zusammengegeben und am Rückfluß gekocht. Gegebenenfalls wird vom Ungelöstem abfiltriert und eingeengt.

Die neuen Verbindungen fallen in kristalliner Form an und werden durch ihren Schmelzpunkt charakterisiert.

Wie bereits mehrfach erwähnt, zeichnen sich die erfindungsgemäßen N,N-Dialkyl-O-pyrimidinyl-carbaminsäureester durch eine hervorragende insektizide Wirksamkeit aus.

Die Wirkstoffe eignen sich bei guter Pflanzenverträglichkeit und günstiger Warmblütertoxizität zur Bekämpfung von tierischen Schädlingen, insbesondere Insekten, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus,

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp.

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalsiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp.,

Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp.

Die Wirkstoffe können in die überlichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate: gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehle, Kokosnußschalen, Maiskolben und Tabakstengel; als Emulgier- und/oder schaumerzeugende Mittel: nichtionogene und anionische Emulgatoren, wie Polyoxyäthylen-Fettsäure-Ester, Polyoxyäthylen-Fettalkohol-Äther, z.B. Alkylaryl-polyglykol-äther, Alkylsulfonate, Alkylsulfate, Arylsulfonate sowie Eiweißhydrolysate; als Dispergiermittel: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganishe Pigmente, z.B. Eisenoxid, Titanoxid, Ferrocyanblau und organische Farbstoffe, wie Alizarin-, Azo-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungsformen angepaßten üblichen Weise.

## Beispiel A

### Myzus-Test (Kontakt-Wirkung)

Lösungsmittel: 3 Gewichtsteile
Emulgator : 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit der Wirkstoffzubereitung werden Kohlpflanzen (Brassica oleracea), welche stark von der Pfirsichblattlaus (Myzus persicae) befallen sind, tropfnaß besprüht.

Nach den angegebenen Zeiten wird die Abtötung in % bestimmt. Dabei bedeutet 100%, daß alle Blattläuse abgetötet wurden; 0% bedeutet, daß keine Blattläuse abgetötet wurden.

Wirkstoffe, Wirkstoffkonzentrationen, Auswertungszeiten und Resultate gehen aus der nachfolgenden Tabelle A hervor:

TABELLE A

(pflanzenschädigende Insekten)

Myzus-Test

| Wirkstoffe | Wirkstoffkonzentration in % | Abtötungsgrad in % nach 1 Tag |
|---|---|---|
| 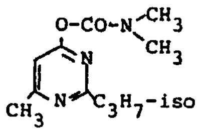 (bekannt) | 0,1<br>0,01<br>0,001 | 100<br>50<br>0 |
| 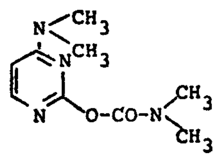 | 0,1<br>0,01<br>0,001 | 100<br>100<br>70 |
| 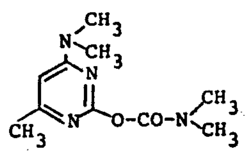 | 0,1<br>0,01<br>0,001 | 100<br>100<br>40 |
| 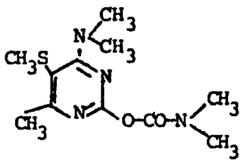 | 0,1<br>0,01<br>0,001 | 100<br>100<br>85 |

Beispiel B
Grenzkonzentrations-Test/Wurzelsystemische Wirkung

Testinsekt: Myzus persicae
Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des

Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100%, wenn alle Testtiere abgetötet sind und 0%, wenn noch genau so viele Testinsekten leben wie bei der unbehandelten Kontrolle.

Wirkstoffe, Aufwandmengen und Resultate gehen aus der nachfolgenden Tabelle B hervor:

TABELLE B

(Wurzelsystemische Wirkung)

Myzus persicae

| Wirkstoffe | Abtötungsgrad in % bei einer Wirkstoffkonzentration von 5 ppm |
|---|---|
| 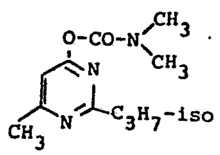 (bekannt) | 0 |
| 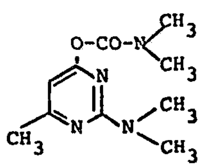 (bekannt) | 0 |
| 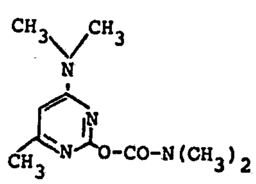 | 100 |
| 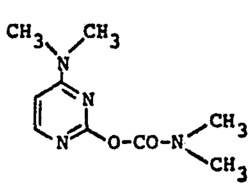 | 100 |

TABELLE B (Fortsetzung)

(Wurzelsystemische Wirkung)

Myzus persicae

| Wirkstoff | Abtötungsgrad in % bei einer Wirkstoffkonzentration von 5 ppm |
| --- | --- |
| 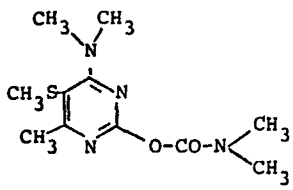 | 100 |
| 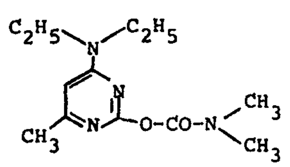 | 100 |

*Herstellungsbeispiele*

Beispiel 1

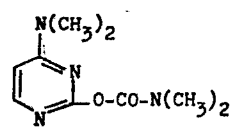

Eine Mischung aus 14g (0,1 Mol) 2-Hydroxy-4-dimethylaminopyrimidin, 16,6g (0,12 Mol) Kaliumcarbonat, 200 ml Chloroform und 12,8 g (0,12 Mol) N,N-Dimethylcarbaminsäurechlorid wird 20 Stunden unter Rückfluß gekocht. Dann filtriert man vom Ungelöstem ab und dampft das Filtrat im Vakuum ein. Zurück bleiben 17,6 g (84% der Theorie) N,N-Dimethyl-O-(4-dimethylamino-pyrimidin(2)yl)carbaminsäureester in Form hellbrauner Kristalle mit dem Schmelzpunkt 92°C.

Analog dem Beispiel 1 können die folgenden Verbindungen der Formel

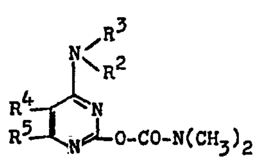

hergestellt werden:

| Beispiel Nr. | $R^2$ | $R^3$ | $R_2$ | $R^5$ | Ausbeute (% der Theorie) | Schmelzpunkt (°C) |
|---|---|---|---|---|---|---|
| 2 | $CH_3$ | $CH_3$ | H | $CH_3$ | 89 | 154 |
| 3 | $CH_3$ | $CH_3$ | Cl | $CH_3$ | 43 | 74 |
| 4 | $CH_3$ | $CH_3$ | $SCH_3$ | $CH_3$ | 82 | 66 |
| 5 | $C_2H_5$ | $C_2H_5$ | H | $CH_3$ | 88 | 70 |
| 6 | $CH_3$ | $CH_3$ | $CH_3$ | $CH_3$ | | |
| 7 | $C_2H_5$ | $C_2H_5$ | H | H | | |

Die als Ausgangsmaterialien zu verwendenden 2-Hydroxy-4-dialkylamino-pyrimidine (II) können z.B. wie folgt hergestellt werden:

Beispiel a:

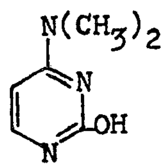

15,8g (0,1 Mol) 2-Chor-4-dimethylamino-pyrimidin (Herstellung siehe J. Chem. Soc. Perkin Trans. II, 1972, Seite 457) werden zusammen mit 16,4g (0,2 Mol) Natriumacetat in 100 ml Eisessig 4 Stunden unter Rückfluß gekocht. Dann destilliert man das Lösungsmittel im Vakuum ab, versetztden Rückstand mit 150 ml Wasser und dampft wieder ein. Der Rückstand wird in 150 ml Wasser gelöst und 3× mit je 100 ml Chloroform extrahiert. Die organischen Phasen werden über Natriumsulfat getrocknet, dann destilliert man das Lösungsmittel im Vakuum ab. Zurück bleiben 9,3g (68% der Theorie) 2-Hydroxy-4-dimethylamino-pyrimidin in Form farbloser Kristalle mit dem Schmelzpunkt 242°C.

Analog Beispiel a) können die folgenden Verbindungen hergestellt werden:

Beispiel b:

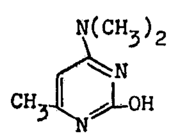

in 88%iger Ausbeute mit dem Schmelzpunkt von 265°C.

Beispiel c:

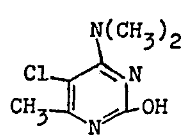

in 51%iger Ausbeute mit dem Schmelzpunkt von 234°C.

Beispiel d:

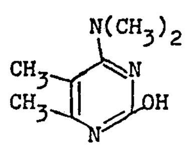

Beispiel e:

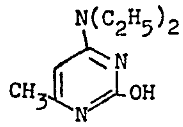

in 89%iger Ausbeute mit einem Schmelzpunkt von 208°C.

Beispiel f:

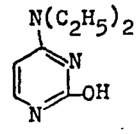

Beispiel g:

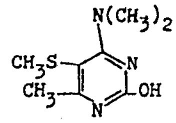

Eine Lösung von 4,7g (0,05 Mol) Dimethyldisulfid in 150 ml Methylenchlorid wird bei —20°C innerhalb von 30 Minuten mit 6,8 g (0,05 Mol) Sulfurylchlorid versetzt. Nach 20 Minuten tropft man diese Lösung bei —10°C zu einer Suspension von 17g (0,1 Mol) 2-Hydroxy-4-dimethylamino-6-methyl-pyrimidin-Natriumsalz in 200 ml Methylenchlorid. Das Gemisch wird dann 18 Stunden bei Raumtemperatur nachgerüht. Anschließend extrahiert man die Lösung mit 200 ml Wasser, trocknet die organische Phase über Natriumsulfat und destilliert das Lösungsmittel im Vakuum ab. Zurück bleibe 8,8 g (45% der Theorie) 2-Hydroxy-4-dimethyl-amino-5-methylthio-6-methyl-pyrimidin in Form farbloser Kristalle mit dem Schmelzpunkt 209°C.

Analog Beispiel g kann hergestellt werden:

Beispiel h:

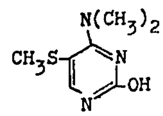

**Patentansprüche**

1. N,N-Dialkyl-O-pyrimidinyl-carbaminsäureester der Formel (I)

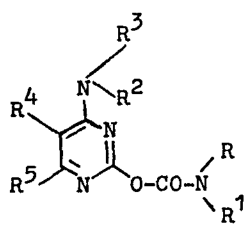

(I)

worin

R bis $R^3$ für gleiches oder verschiedenes Alkyl,
$R^4$ für Wasserstoff, Alkyl, Alkylthio oder Halogen und
$R^5$ für Wasserstoff oder Alkyl stehen.

2. Verfahren zur Herstellung der N,N-Dialkyl-O-pyrimidinylcarbaminsäureester (I), dadurch gekennzeichnet, daß man 2-Hydroxy-4-dialkylamino-pyrimidine der Formel II

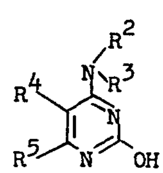

(II)

in welcher

$R^2$ bis $R^5$ die in Anspruch 1 angegebene Bedeutung haben,

a) gegebenenfalls in Form der Alkali- oder Erdalkalisalze oder gegebenenfalls in Gegenwart eines Säureakzeptors mit N,N-Dialkyl-carbaminsäurehalogeniden der Formel (III)

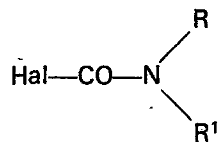

(III)

in welcher
R und $R^1$ die in Anspruch 1 angegebene Bedeutung haben und
Hal für Halogen, vorzugsweise Chlor, steht,
gegebenenfalls in Gegenwart eines Lösungsmittels umsetzt, oder

b) gegebenenfalls in Gegenwart eines Verdünnungsmittels mit Phosgen umsetzt und anschließend mit einem Amin der allgemeinen Formel (IV)

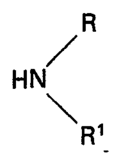

(IV)

in welcher
R und $R^1$ die in Anspruch 1 angegebene Bedeutung haben,
gegebenenfalls in Gegenwart eines Säureakzeptors umsetzt.

3. Insektizide Mittel gekennzeichnet durch einen Gehalt an N,N-Dialkyl-O-pyrimidinyl-carbaminsäureestern gemäß Anspruch 1.

4. Verwendung von N,N-Dialkyl-O-pyrimidinyl-carbaminsäureestern gemäß Anspruch 1 zur Bekämpfung von Insekten.

5. Verfahren zur Herstellung insektizider Mittel, dadurch gekennzeichnet, daß man N,N-Dialkyl-O-pyrimidinyl-carbaminsäureester gemäß Anspruch 1 mit Streckmitteln und/oder oberflächenaktiven Stoffen vermischt.

## Claims

1. N,N-Dialkyl-O-pyrimidinyl-carbamic acid esters of the formula (I)

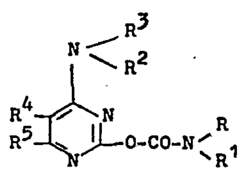

(I)

wherein
R to $R^3$ represent identical or different alkyl,
$R^4$ represents hydrogen, alkyl, alkylthio or halogen and
$R^5$ represents hydrogen or alkyl.

2. Process for the preparation of the N,N-dialkyl-O-pyrimidinyl carbamic acid esters (I), characterised in that 2-hydroxy-4-dialkylamino-pyrimidines of the formula (II)

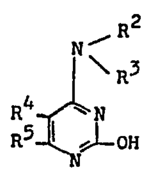

(II)

in which
$R^2$ to $R^5$ have the meaning mentioned in Claim 1

a) are reacted, if appropriate in the form of the alkali metal salts or alkaline earth metal salts or, if appropriate, in the presence of an acid acceptor, with N,N-dialkyl-carbamic acid halides of the formula (III)

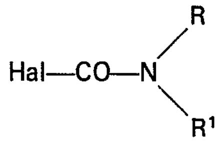

(III)

in which
R and $R^1$ have the meaning mentioned in Claim 1 and Hal represents halogen, preferably chlorine, if appropriate in the presence of a diluent, or

b) are reacted, if appropriate in the presence of a diluent, with phosgene, and subsequently with an amine of the general formula (IV)

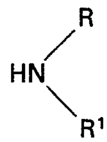

(IV)

in which
R and $R^1$ have the meaning mentioned in Claim 1 and
if appropriate in the presence of an acid acceptor.

3. Insecticidal agents, characterised in that they contain N,N-dialkyl-O-pyrimidinyl-carbamic acid esters according to Claim 1.

4. Use of N,N-dialkyl-O-pyrimidinyl-carbamic acid esters according to Claim 1 for combating insects.

5. Process for the preparation of insecticidal agents, characterised in that N,N-dialkyl-O-pyrimidinyl-carbamic acid esters according to Claim 1 are mixed with extenders and/or surface-active compounds.

**Revendications**

1. Esters d'acides N,N-dialkyl-O-pyrimidinyl-carbamiques de formule (I):

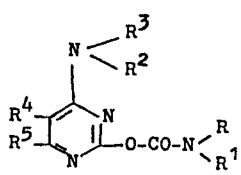

(I)

dans laquelle
R à $R^3$ représentent des groupes alkyle identiques ou différents,
$R^4$ représente un atome d'hydrogène, un groupe alkyle, un groupe alkylthio ou un atome d'halogène, et
$R^5$ représente un atome d'hydrogène ou un groupe alkyle.

2. Procédé de préparation d'esters d'acides N,N-dialkyl-O-pyrimidinyl-carbamiques (I), caractérisé en ce qu'on fait réagir des 2-hydroxy-4-dialkylaminopyrimidines de formule (II):

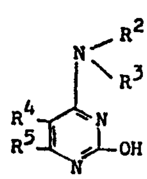

(II)

dans laquelle
$R^2$ à $R^5$ ont les significations indiquées dans la revendication 1,

a) éventuellement sous forme de leurs sels alcalins ou alcalino-terreux ou éventuellement en présence d'un accepteur d'acide avec des halogénures d'acides N,N-dialkyl-carbamiques de formule (III):

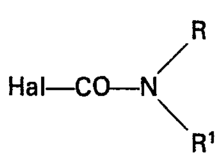

(III)

dans laquelle
R et $R^1$ ont les significations indiquées dans la revendication 1, et

Hal représente un atome d'halogène, de préférence, un atome de chlore, éventuellement en présence d'un solvant, ou

b) éventuellement en présence d'un diluant avec du phosgène, puis avec une amine de formule générale (IV):

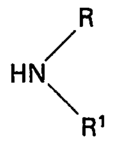

(IV)

dans laquelle

R et $R^1$ ont les significations indiquées dans la revendication 1,

éventuellement en présence d'un accepteur d'acide.

3. Produits insecticides, caractérisés en ce qu'ils contiennent des esters d'acides N,N-dialkyl-O-pyrimidinyl-carbamiques suivant la revendication 1.

4. Utilisation d'esters d'acides N,N-dialkyl-O-pyrimidinyl-carbamiques suivant la revendication 1 pour combattre les insectes.

5. Procédé de préparation de produits insecticides, caractérisé en ce qu'on mélange des esters d'acides N,N-dialkyl-O-pyrimidinyl-carbamiques suivant la revendication 1 avec des diluants et/ou des substances tensio-actives.